# EUROPEAN PATENT SPECIFICATION

(11) **EP 1 705 250 B1**
(45) Date of publication and mention of the grant of the patent: **26.06.2013**
(21) Application number: 03782063.6
(22) Date of filing: 19.12.2003
(51) Int. Cl.: C08G 63/88

(54) **A METHOD FOR SEPARATING, EXTRACTING AND PURIFYING POLY-BETA-HYDROXYALKANOATES (PHAS) DIRECTLY FROM BACTERIAL FERMENTED BROTH**
VERFAHREN ZUR TRENNUNG, EXTRAKTION UND REINIGUNG VON POLY-BETA-HYDROXYALKANOATEN (PHAS) DIREKT AUS DER BAKTERIENFERMENTATIONSBRÜHE
PROCEDE DE SEPARATION, D'EXTRACTION ET DE PURIFICATION DE POLY-BETA-HYDROXYALCANOATES (PHA) DIRECTEMENT A PARTIR D'UN MILIEU DE CULTURE BACTERIEN FERMENTE

(43) Date of publication of application: 27.09.2006
(73) Proprietor: Tianan Biologic Material Co., Ltd. Ningbo, Zhejiang 315800 (CN)
(72) Inventor: CHEN, Xuejun, Tianan Biologic Material Co., Ltd., Zhejiang 315800 (CN)
(74) Representative: Schlief, Thomas P.
(86) International application number: PCT/CN2003/001092
(87) International publication number: WO 2005/059153

(56) References cited:
- WO-A-96/25452
- CN-A- 1 171 410
- CN-A- 1 190 674
- CN-A- 1 328 160
- CN-A- 1 464 063
- DE-A1- 19 712 702
- GB-A- 2 120 671
- JP-A- 2001 046 094
- JP-A- 2001 057 895
- US-A- 4 910 145
- US-A- 5 391 708
- US-A- 5 899 339
- CHOI J.I. ET AL: 'Efficient economical recovery of poly(3-hydroxybutyrate) from recombinant Eschericha coli by simple digestion with chemicals' ADVANCED INSTITUTE OF SCIENCE AND TECHNOLOGY vol. 62, no. 5, 1999, pages 546 - 553, XP002979947

## Description

### Technical field

This invention relates to post-treatment of biological engineering, particularly to extraction and separation of bacterial fermentation product, or more particularly to extraction and separation of polyhydroxyalkanoates in cells.

### Background of invention

Poly- β -hydroxyalkanoates (PHAs) are biological polyesters accumulated in cells by special microorganisms under special growth conditions.

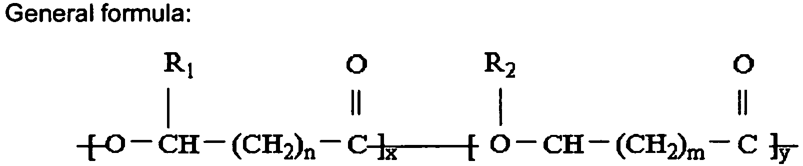

In which, n and m are 1~4 integer, usually 1, that is 3-hydroxyalkanoates (3-HAS); R₁ and R₂ are straight chain or branched chain C₁₋₁₂ alkyl which are substituted or non-substituted; X and Y are not 0 simultaneously, and determine the content of the component in copolymer. The average molecular weight of PHAs is generally 1-4 million Da.

The physical property of PHAs is similar to that of polypropylene. As its biodegradability, biocompatibility, piezoelectricity and optical activity are characteristics not possessed by common petrochemical resins, it has wide application prospect in industry, agriculture, medicine, sanitation, food, electronics, etc.

Large scale industrialized production of PHAs has not been realized internationally. The principal reason is the cost is much higher than that of petrochemical resin. The cost of PHAs includes mainly material cost and separation purification cost. The material cost depends on production efficiency of bacteria species and fermentation technology, whilst the separation purification cost depends largely on technology. The current extraction technology includes separation of cells from fermentation liquid with high speed centrifuge and purification of PHAs in separated wet bacterial body with organic solvent extraction, chemical reagent or surfactant + enzyme. These methods have the defect of high cost or serious pollution, and are difficult to be industrialized. One step extraction separation method for extracting polyhydroxyalkanoates directly from fermentation liquid containing cells is disclosed in Chinese patent application CN-A-1 328 160, but it must use large quantity of sodium hypochlorite and has the defect of poor operation environment, serious pollution, cost increased by waste water treatment, and product quality affected by shear degradation of PHAs.

A further method for separating hydroxybutyrates is disclosed by GB-A-2 120 671. Hydroxybutyrates polymers are separated from solutions thereof by causing the solution to gel and then shearing the gel, and removing any expressed solvent, to give discrete gel particles free of any continuous liquid phase. Solvent is then evaporated from the discrete particles e.g. in a fluid bed drier.

According to WO 96/25452 A a process of producing a polyhydroxyalkanoates PHA latex first comprises the steps of making a liquid-form solution of such PHA in a water soluble liquid and contacting that solution with water under shear. Suitably, the solution is prepared at a temperature between the boiling point of water and 10° C below the melting point of the PHA, for example by melt separating PHA from a microbiological suspension thereof, dissolving it in the water soluble liquid, and then contacting the solution at over 100°C with water at 50-95°C. The water-soluble liquid may be for example 1,2-propandiol or a liquid more volatile than water such as ethanol, propanol or tertiary butanol. A surfactant may be present or added after contacting, for example one providing steric stabilization such as an acrylic graft copolymer emulsifier.

Another process for deriving polyhydroxyalkanoates is presented by DE 197 12 702 A1. In a first step, cells of microorganisms containing polyhydroxyalkanoates are exposed to mechanical forces and separated from the remaining components in an aqueous environment. After a drying step, soluble components are dissolved by means of solvents. The final derivation of the remaining polyhydroxyalkanoates from the residue is realized thereafter by means of extraction, precipitation and washing according to standard procedures.

The process according to US-A-5 899 339, also relating to a process for recovering polyhydroxyalkanoate from biological source material comprises the steps of comminuting the biological source material, suspending the comminuted biological source material in a fluid, partitioning the polyhydroxyalkanoate from the other components of the biological source material by centrifugal fractionation to form a solid-solid separation, and recovering the polyhydroxyalkanoate.

In accordance with the disclosure of CN-A-1 190 674, another method of separating and purifying endocellular poly-β-hydroxyalkanoates from bacteria thallus includes the steps of treating the bacteria thallus obtained from fermentation with anionic surfactant under alkaline condition and centrifugal extracting the contained poly-β-hydroxyalkanoates, treating the poly-β-hydroxyalkanoates product obtained with proteinase, and collecting and drying the obtained poly-β-hydroxyalkanoates product.

A further method for extracting poly-β-hydroxyalkanoates, especially from A. eutrophus, is disclosed by CN-A-1 171 410. The poly-β-hydroxyalkanoates is extracted in a single step from A. eutrophus with a aqueous solution of a surfactant and a complexant.

The production of poly-3-hydroxyalkanoates as being disclosed by US-A-5 391 708 entails polymerizing one or more β-substituted-β-propiolactone(s) under polymerization conditions in the substantial absence of water and in the presence of an anionic initiator.

Another method for recovering of microbial poly-3-hydroxybutyrate is disclosed by Choi J.I. et al (Efficient economical recovery of poly(3-hydroxybutyrate) from recombinant Escherichia coli by simple digestion with chemicals; Advanced institute of science and technology; vol. 62, no. 5, 1999, pages 546-553). To optimize digestion conditions, NaOH and KOH were used. In addition, different acids, alkalies, and surfactants were examined for their ability to digest non-P(3HB) cellular materials (NPCM).

Following the instructions of US-A-4 910 145, an aqueous suspension of micro-organism cells containing a 3-hydroxyalkanoate polymer are subjected to a proteolytic enzyme digestion and/or a surfactant digestion in order to solubilise cell material other than the 3-hydroxyalkanoate. Prior to or during the digestion, but before any proteolytic enzyme digestion step, the suspension is heated to at least 80 °C to denature nucleic acids which otherwise hinder separation of the 3-hydroxyalkanoate polymer containing residue from the suspension.

To separate and purify PHA in accordance with JP 2001 046094 A, a surfactant is added to a suspension of a microbial cell of a microorganism (e.g. Aeromonas caviae), containing a poly-3-hydroxyalkanoic acid comprising a biopolymer of D-3-hydroxyhydroxybutyrate and D-3-hydroxyhexanoate, and a terpolymer of the D-3-hydrobutyrate, D3-hydroxyvalerate and D-3-hydroxy-hexanoate. The resultant mixture liquid is then subjected to a physical crushing treatment to thereby separate and purify the objective poly-3-hydroxyal-kanoic acid suitable as a raw materials, etc. for a plastic product, a drug carrier, a fertilizer carrier, an agricultural mulching film, a fishing gear such as a fishing line, a bag, such as a refuse bag, or for composts in high purity and high yield.

According to the method disclosed by JP 2001 057895 A a divalent or polyvalent metal salt (e.g. calcium chloride), and/or a surfactant (e.g. benzyltrimethylammonium chloride) is added to a suspension of a microbial cell of poly-3-hydroxyalkanoic acid (PHA)-containing microorganism (e.g. Aicali-genes eutrophus A32C (FERM P-15786) strain into which a PHA synthase gene derived from Aeromonas caviae is transferred) and an extraction solvent (e.g. chloroform). Undissolved cell residue is flocculated and removed from the PHA-containing solution to readily obtain a high-purify poly-3-hydroxyalkanoic acid useful as a biodegradable plastic.

Finally, CN-A-1 464 063 suggests a process of directly separating and purifying intracellular polyhydroxy fatty acid enzymes from bacterial fermented liquid. The fermented liquid is first treated physically to break cell wall, then pH regulated to become alkaline and mixed with anionic surfactant and coagulant via stirring, and finally precipitant separated, with the precipitant being washed and dried.

The purpose of this invention is to provide an extraction method for PHAs, which can reduce effectively separation and purification cost, reduces pollution, and is suitable for industrialized production.

### Invention

This invention provides a method for directly separating and purifying polyhydroxyalkanoates in cells from bacterial fermentation liquid, comprising:
(1) pretreating fermentation liquid with physical method for breaking cell wall;
(2) adjusting the pH value of the pretreated fermentation liquid so that it is alkaline;
(3) adding anionic surfactant and agitating;
(4) separating and extracting precipitate in reaction liquid;
(5) washing and drying;
wherein the physical method includes mechanical breaking or ultrasonic breaking; the mechanical breaking includes ball milling or high pressure homogenization; the sequence of adjusting pH and adding surfactant is interchangeable.

In step 3, aside from adding anionic surfactant, coagulating agent can be added.

The physical method used to break cell wall can be ultrasonic breaking, ball milling or high pressure treatment.

The pH of the pretreated fermentation liquid is adjusted to 8-13. The alkaline substance used in adjusting pH can be solid or aqueous solution of NaOH, Na₂CO₃, NaHCO₃ or ammonia water.

The anionic surfactant can be olefinesulfonate (AOS), fatty alcohol sulfate, fatty alcohol polyoxyethylene-ether sulfate (AES), fatty alcohol-polyoxyethylene ether (AEO), alkylphenol-polyoxyethylene ether, etc., its quantity is 0.5-20 % (WN) of fermentation liquid.

The coagulating agent is sodium polyacrylate, modified starch, polyamine, etc., its quantity is 0.5-20 % (WN) of the fermentation liquid.

After adding the anionic surfactant and the coagulating agent, the reaction temperature under agitation is 10-70 °C and the reaction time 5-60 min.

Centrifuge, filter-press, vacuum suction filtration, etc. can be used for separating and extracting precipitate from the reaction liquid.

The invention is applicable to separation and purification of fermentation liquid of bacteria and its aberrance and gene engineering bacteria containing polyhydroxyalkanoates. Applicable bacteria include Alcaligenes, Pseudomonas, Azotobacter, Rhodospirillum, Methylotrophs, Bacillus, etc.

The invention has no high requirement for dry weight of cells and content of PHAs in fermentation liquid. The invention has the advantage of simple technology, low cost, high yield and greatly reduced pollution, so large scale industrialized production can be realized.

### Detailed description of the invention

Following examples are used to further describe the invention. These examples should not constitute any limitation to the scope of claims. Any modifications or changes made by the skilled man in the art benefit from the disclosure of this application should be included within the scope of claims stated in this application.

### Example 1

Take 1000ml of fermentation liquid of Alcaligenes entrophus mutant 65-7, in which the dry weight of cells is 142 g/l, the content of PHBV is 78.5 %; pretreat with ball mill (530 r/min, 0.1 mm steel ball) for 40 min; adjust pH value to 12 with 30 % NaOH solution; add 13 g of sodium laurylsulfate; adjust reaction temperature to 32 °C; react under agitation for 5 min; filter with suction and filter paper; wash precipitate with water till washing becomes neutral; dry at 70 °C to constant weight. Purity of the product is 98.2 %, the average molecular weight is 5.2x10⁵ Da, the yield is 85.2 %. the COD and BOD of waste water from suction filtration after treatment with anaerobic and aerobic bacteria is 800 and 30 mg/l respectively, in conformity with state discharge standard.

### Example 2

Take 100 ml of fermentation liquid of Alcaligenes entrophus, in which the dry weight of cells is 147 g/l, the content of PHBV is 75.2 %; break cell wall with ultrasonic (1500 W) for 20 min; adjust pH value to 8 with 30 % NaOH solution; add 0.5 g of sodium laurylsulfate and 5 g of sodium polyacrylate; adjust reaction temperature to 70 °C; react under agitation for 30 min; filter with suction and filter paper; wash coagulated precipitate with water till washing becomes neutral; dry in oven at 70 °C to constant weight. Purity of the product is 93.2 %, the average molecular weight is 4.1x10⁵ Da, the yield is 80.3 %.

### Example 3

Take 50 ml of fermentation liquid of Alcaligenes entrophus, in which the dry weight of cells is 102 g/l (in which the content of PHB is 60 %); pretreat with ball mill (560 r/min, 0.1 mm steel ball) for 30 min; adjust pH value to 13 with NH₃.H₂O solution; add 10 g of sodium laurylsulfate and 10 g of modified starch; adjust reaction temperature to 10 °C; react under agitation for 10 min; separate with centrifuge (separation factor 600); wash precipitate with water till washing becomes neutral; dry in oven at 70 °C to constant weight. Purity of the product is 98.2 %, the average molecular weight is 4.4x10⁵ Da, the yield is 87 %.

### Example 4

Take 500 ml of fermentation liquid of Alcaligenes entrophus mutant 65-7, in which the dry weight of cells is 135 g/l, the content of PHB is 75.5 % and introduce it into a special vessel. Increase pressure to 60 MPa, release pressure rapidly after 10 min, collect the liquid and repeat the operation twice. Adjust pH value to 10 with 30 % NaOH solution; add 9 g of sodium lauryl polyoxyethylene ether sodium sulfate; adjust reaction temperature to 38 °C; react under agitation for 8 min; filter with suction and filter paper; wash precipitate with water till washing becomes neutral; dry at 70 °C to constant weight. Purity of the product is 96.7 %, the average molecular weight is 4.2x10⁵ Da, the yield is 81.5 %.

### Example 5

Take 100 ml of fermentation liquid of Alcaligenes entrophus, in which the dry weight of cells is 154 g/l (in which content of PHBV is 80.5 %); break cell wall with ultrasonic (2800 W, continuous treatment) for 40 min; adjust pH value to 11 with 30 % NaOH solution; add 10 kg of sodium laurylsulfate and 0.5 kg of sodium polyacrylate; adjust reaction temperature to 50°C; react under agitation for 60 min; filter with filter press; wash precipitate with water till washing becomes neutral; dry in oven at 70 °C to constant weight. Purity of the product is 97 %, the average molecular weight is 5.3x10⁵ Da, the yield is 84 %.

### Example 6

Take 100 ml of fermentation liquid of Pseudomonas, in which dry weight of cells is 86 g/l, content of PHBV is 61.5 %; pretreat with ball mill (560 r/min, 0.1 mm steel ball) for 50 min; adjust pH value to 11 with 30 % NaOH solution; add 3 g of sodium laurylsulfate; adjust reaction temperature to 24 °C; react under agitation for 10 min; filter with suction and filter paper; wash precipitate with water till washing becomes neutral; dry at 70 °C to constant weight. Purity of the product is 94.2 %, the average molecular weight is 3.2x10⁵ Da, the yield is 71.2 %.

## Claims

1. A method for directly separating and purifying polyhydroxyalkanoates in cells from bacterial fermentation liquid, comprising:
(1) pretreating of the fermentation liquid with physical method to break cell wall;
(2) adjusting pH value of the pretreated fermentation liquid to alkaline;
(3) adding anionic surfactant and reacting under agitation;
(4) separating and extracting coagulated precipitate from the reaction liquid;
(5) washing and drying;
wherein the physical method includes mechanical breaking or ultrasonic breaking; the mechanical breaking includes ball milling or high pressure homogenization; the sequence of adjusting pH and adding surfactant is interchangeable.

2. The method according to claim 1, wherein coagulating agent can be added in the step (3).

3. The method according to claim 1, wherein the mechanical breaking is ball milling.

4. The method according to claim 1, wherein pH of the pretreated fermentation liquid is adjusted to 8-13.

5. The method according to claim 1 or 4, wherein the alkaline substance used to adjust pH is NaOH, Na₂CO₃, NaHCO₃ solid/aqueous solution or ammonia water.

6. The method according to claim 1, wherein the anionic surfactant is olefin sulfonate, fatty alcohol sulfate, fatty alcohol polyoxyethylene ether sulfate, fatty alcohol polyoxyethylene ether or alkylphenol polyoxyethylene ether, its quantity is 0.5-20 % (WN) of the fermentation liquid.

7. The method according to claim 1, wherein the coagulating agent is selected from sodium polyacrylate, modified starch or polyamine, its quantity is 0.5-20 % (WN) of the fermentation liquid.

8. The method according to claim 1, wherein the reaction temperature under agitation is 10-70 °C, the time is 5-60 min.

9. The method according to claim 1, wherein the method for separating and extracting coagulated precipitate from reaction liquid is selected from centrifuge, filter press or vacuum suction filtration.

## Patentansprüche

1. Verfahren zum direkten Trennen und Läutern von Polyhydroxyalkanoaten in Zellen aus bakterieller Fermentationsflüssigkeit, umfassend:
(1) das Vorbehandeln der Fermentationsflüssigkeit mit physikalischem Verfahren, um die Zellwand aufzubrechen;
(2) das Einstellen des pH-Werts der vorbehandelten Fermentationsflüssigkeit auf alkalisch;
(3) das Zufügen eines anionischen Netzmittels und Reagieren unter Rühren;
(4) das Trennen und Extrahieren des koagulierten Bodenkörpers aus der Reaktionsflüssigkeit;
(5) das Waschen und Trocknen;
wobei das physikalische Verfahren mechanisches Aufbrechen oder Ultraschall-Aufbrechen umfasst; wobei das mechanische Aufbrechen Kugelmahlen oder Hochdruck-Homogenisierung umfasst; wobei die Abfolge des Einstellens des pH-Werts und des Zufügens des Netzmittels untereinander austauschbar ist.

2. Verfahren nach Anspruch 1, wobei Koaguliermittel im Schritt (3) zugefügt werden kann.

3. Verfahren nach Anspruch 1, wobei das mechanische Aufbrechen Kugelmahlen ist.

4. Verfahren nach Anspruch 1, wobei der pH-Wert der vorbehandelten Fermentationsflüssigkeit auf 8-13 eingestellt ist.

5. Verfahren nach Anspruch 1 oder 4, wobei die zum Einstellen des pH-Werts verwendete alkalische Substanz NaOH, Na₂CO₃, NaHC0₃ als Feststoff/wässerige Lösung oder Salmiakgeist ist.

6. Verfahren nach Anspruch 1, wobei das anionische Netzmittel Olefinsulfonat, Fettalkoholsulfat, Fettalkohol-Polyoxyethylenethersulfat, Fettalkohol-Polyoxyethylenether oder Alkylphenol-Polyoxyethylenether ist und seine Menge 0,5-20 % (Masse/Vol) der Fermentationsflüssigkeit beträgt.

7. Verfahren nach Anspruch 1, wobei das Koaguliermittel aus Natriumpolyacrylat, modifizierter Stärke oder Polyamin gewählt ist und seine Menge 0,5-20 % (Masse/Vol) der Fermentationsflüssigkeit beträgt.

8. Verfahren nach Anspruch 1, wobei die Reaktionstemperatur während des Rührens 10-70 °C, die Zeit 5-60 min beträgt.

9. Verfahren nach Anspruch 1, wobei das Verfahren zum Trennen und Extrahieren des koagulierten Bodenkörpers aus der Reaktionsflüssigkeit aus Zentrifugen-, Pressfilter- und Vakuumsaugfiltrierung gewählt ist.

## Revendications

1. Méthode pour séparer directement et purifier des polyhydroxyalcanoates dans des cellules d'un liquide de fermentation bactérienne, comprenant :
(1) le prétraitement du liquide de fermentation par une méthode physique afin de décomposer les parois cellulaires ;
(2) le réglage, à une valeur alcaline, de la valeur de pH du liquide de fermentation prétraité ;
(3) l'addition d'un agent de surface anionique et la réaction par agitation ;
(4) la séparation et l'extraction du précipité coagulé depuis le liquide de réaction ;
(5) le lavage et le séchage ;
dans laquelle la méthode physique comprend la décomposition mécanique ou la décomposition ultrasonique ; la décomposition mécanique comprend le broyage à billes ou l'homogénéisation à haute pression ; la séquence de réglage du pH et d'addition de l'agent de surface est interchangeable.

2. Méthode selon la revendication 1, dans laquelle un agent de coagulation peut être ajouté à l'étape (3).

3. Méthode selon la revendication 1, dans laquelle la décomposition mécanique est le broyage à billes.

4. Méthode selon la revendication 1, dans laquelle le pH du liquide de fermentation prétraité est réglé à une valeur de 8-13.

5. Méthode selon la revendication 1 ou 4, dans laquelle la substance alcaline utilisée pour régler le pH est du NaOH, du Na₂CO₃, une solution solide/aqueuse de NaHC0₃ ou de l'eau ammoniacale.

6. Méthode selon la revendication 1, dans laquelle l'agent de surface anionique est du sulfonate d'oléfine, du sulfate d'alcool gras, du sulfate d'éther d'alcool gras polyoxyéthyléné ou de l'éther d'alkylphénol polyoxyéthyléné, sa quantité est de 0,5-20 % (poids/volume) du liquide de fermentation.

7. Méthode selon la revendication 1, dans laquelle l'agent de coagulation est sélectionné entre le polyacrylate de sodium, l'amidon modifié ou la polyamine, sa quantité est de 0,5-20 % (poids/volume) du liquide de fermentation.

8. Méthode selon la revendication 1, dans laquelle la température de réaction sous agitation est de 10-70 °C, la durée est de 5-60 min.

9. Méthode selon la revendication 1, dans laquelle la méthode pour séparer et extraire le précipité coagulé depuis le liquide de réaction est sélectionnée entre la filtration par centrifugeuse, la filtration par filtre-presse et la filtration par succion sous vide.
